# EUROPEAN PATENT SPECIFICATION

(11) **EP 2 533 838 B1**
(45) Date of publication and mention of the grant of the patent: **10.06.2020**
(21) Application number: 11741743.6
(22) Date of filing: 04.02.2011
(51) Int. Cl.: A61M 16/06

(54) **QUICK RELEASE HEADGEAR STRAP**
SCHNELL ABNEHMBARER KOPFHÖRERBÜGEL
SANGLE DE CASQUE À LIBÉRATION RAPIDE

(30) Priority: 12.02.2010 AU 2010900563
(43) Date of publication of application: 19.12.2012
(73) Proprietor: Compumedics Medical Innovation Pty Ltd, Abbotsford, Victoria 3067 (AU)
(72) Inventor: ZIV, Hedi, Abbotsford Victoria 3067 (AU); SLABBERT, Rikus, Abbotsford Victoria 3067 (AU); PIERI, Alexander, Abbotsford Victoria 3067 (AU)
(74) Representative: Handsome I.P. Ltd
(86) International application number: PCT/AU2011/000103
(87) International publication number: WO 2011/097668

(56) References cited:
- WO-A1-2010/135785
- DE-A1- 19 947 722
- DE-A1- 19 947 722
- GB-A- 2 190 299
- GB-A- 2 190 299
- US-A- 1 958 911
- US-A- 5 630 254
- US-A1- 2003 172 445
- US-A1- 2003 172 445
- US-A1- 2003 178 026
- US-A1- 2003 178 026
- US-A1- 2009 078 266

## Description

This application claims the priority and benefit of Australian Provisional Application No. 2010900563, filed on 12 February, 2010.

The present invention relates to straps used in headgear for masks for the provision of gases to subjects, In particular, masks for gas delivery under pressure to the airways of a subject.

Some treatments require the use of a mask assembly for the delivery of gas or air to a subject. For example, in the treatment of sleep apnoea, gas is often delivered at continuous positive airway pressure (CPAP) wherein gas is supplied continuously at a pressure greater than ambient, or variable positive airway pressure (VPAP) wherein gas is supplied at varying pressures, or other such methods such as BiPAP wherein gas is supplied at two pressures, or APAP wherein gas is supplied at pressures determined automatically by the delivery system to a sleeping subject through a mask to keep the patient's airways open for effective respiration. Often, gas must be delivered through a mask for sustained time periods, for example, through the whole, overnight period of sleep.

It is important for continuous therapeutic benefit from pressurised gas or air that a mask assembly be comfortable and relatively leak-proof. The mask must be comfortable so that a subject achieves the therapeutic benefit of relatively unbroken sleep periods. Masks known in the art often comprise of an interface such as a cushion or pillow, made of soft material to be most comfortable for a subject during long periods of use, but are capable of sealing adequately to minimise leaks. In mask assemblies known in the art, the masks are often fitted on a user's face or nose and held in place by a headgear comprising of fully or partially- elastic straps,

Prior art mask assemblies and their harnessing straps are fitted individually to a subject to obtain an optimal setting or configuration. An optimal setting is one which minimises discomfort while delivering gas with minimal leakage.

Prior strap assemblies including locking means are described in US20030178026A1, GB2190299, US2003172445, US2009078226, US1958911 and US5630254. DE19947722 describes a respiratory mask support which includes means for setting an optimal length including a second strap.

A common user complaint with current masks is that it is complex to re-fit a mask after taking it off and ensuring the mask configuration is optimal on re-fitting. What is needed is a mask assembly incorporating a headgear with more effective retention of optimal mask configuration during repeated use.

It is an object of the invention to provide a headgear for a mask assembly that is relatively quick and easy to release and quick and easy to re-fit in repeated use while maintaining a comfortable and relatively leak-proof configuration. The invention provides a means for engaging a mask frame that provides solutions for two problems with prior art engagement means. The invention is defined by the independent claims.

In a first aspect, it is defined a headgear for a mask assembly for the provision of gases to subjects, the headgear comprising a strap for positioning the mask assembly on a user's head; the mask assembly including an aperture through which the strap is passable, in use; the strap including first and second adjacent portions, the second portion being folded against the first portion, in use, wherein the second portion is threadable through the aperture; the first and second portions each including a surface for engagement with the complementary surface of the other adjacent portion; characterised in that the headgear further comprises a strap length delimiting means on the first portion that moves in a sliding action along an axis of the strap such that the strap length delimiting means is adjustable in relation to the strap, but has enough friction or resistance to prevent undesired movement, and is thicker or longer than the aperture such that, in use, the strap length delimiting means acts as an optimal setting memory which operates when the user pulls the second portion of the strap while putting the mask assembly on and fastening the straps to prevent the first portion from sliding past an optimal setting marker when the strap length delimiting means comes into contact with the aperture.

The length delimiting means may be a locking mechanism for preventing undesired movement while enabling a sliding feature for optimal configuration. The locking mechanism may comprise a spring-based stopper. The headgear may further comprise another strap delimiting means having a stud or a lug or a thickening of the second portion of the strap.

Preferably, the length-delimiting means employs friction to remain in place. The strap surfaces may be modified to provide the delimiting means. Preferably, the strap the length delimiting means is in engagement with the strap. Other means for delimiting the effective strap length may be used.

In another aspect, it is defined a method for positioning the headgear for a mask assembly for the provision of gases to subjects according to the first aspect, comprising the steps of: placing the headgear on the subject's head; fitting said headgear; and adjusting the fit of said headgear with a length delimiting means.
Figure 1 shows a side perspective example of a prior art headgear and mask assembly having a strap interface.
Figure 2 shows a perspective view of an embodiment of a strap of a headgear for a mask assembly according to the invention.
Figure 3 shows a perspective view of another embodiment of a strap of a headgear for a mask assembly according to the invention.

The appended figures illustrate embodiments of the invention. It will be understood that there are many other possible embodiments of the invention and that the invention is limited only by the scope of the claims appended hereto.

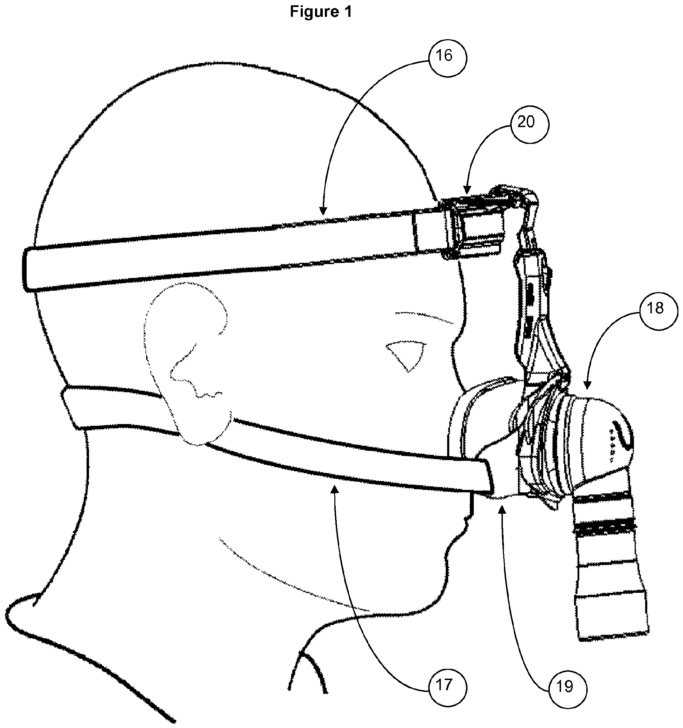

Figure 1 shows a prior art headgear and mask assembly for positioning a nasal mask for gas delivery to a subject. The headgear comprises of straps, a mask 18 for sealing a nasal cushion against the face of a subject, the mask including a frame 19 for engaging a headgear comprising of straps 16, 17. The mask 18 is usually fitted to the user's face by at least two straps 16 & 17 in various arrangements known in the art. It will be understood that the straps 16, 17 may each encircle the head as a single strap or each strap 16, 17 comprise of a pair of straps of varying material composition and resilience, one of each pair of straps on a side of the face or head of a subject. A strap 16, 17 may engage the mask frame 19 in various methods. The present invention provides advanced solutions for headgear straps and their engagement with the mask frame or extensions thereof, such as a forehead support (such as 20 in Figure 1), or as a mask frame (such as 19 in Figure 1).

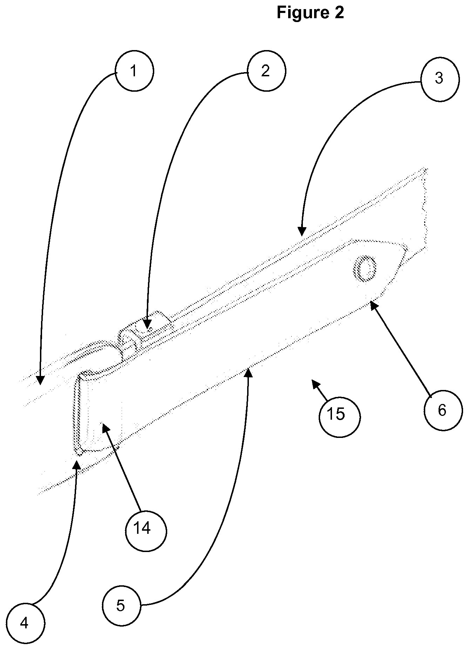

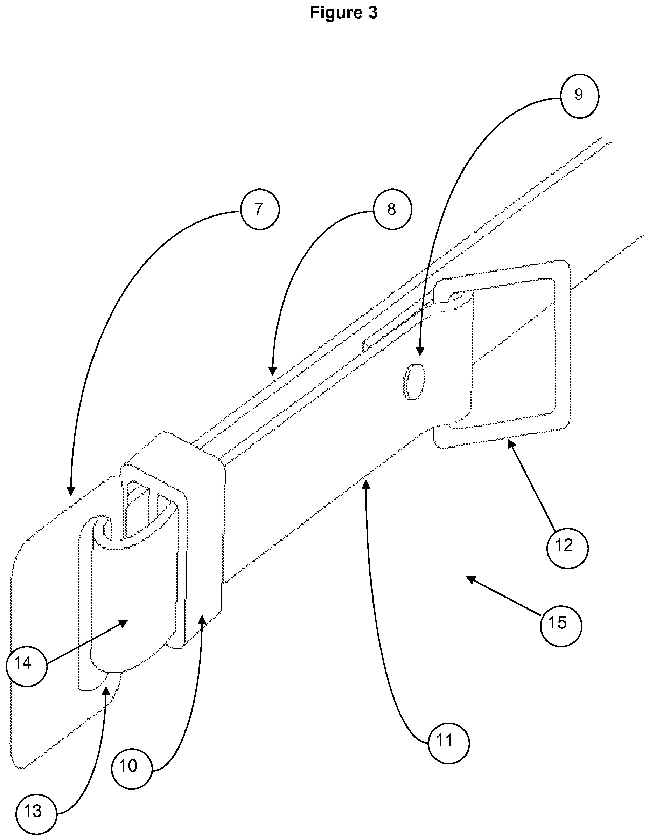

Figures 2 and 3 show embodiments of the invention as a strap 15 (being either or both of straps 16 or 17 of Figure 1) comprising of a first portion 3 & 8 a second portion 5 & 11 and a third portion 14, the second portions being folded adjacent the first portions, the third portion engaged with a mask. Each of the first and second portions may include a surface for engagement with the complementary surface of the other adjacent portion. The third portion 14 engages a mask from a mask assembly (such as 19 or 20 in Figure 1). The strap 15 ma comprise of any suitable material, which may be elastic or inelastic. The second portion 5, 1 1 is threaded through an aperture or slot (4 Figure 2 or 13 Figure 3) in a mask frame or attachment (1 Figure 2 or 7 Figure 3) characteristically used in a mask assembly so that the third portion 14 engages the mask frame (4 Figure 2 or 13 Figure 3). The second portions 5, 1 1 of the strap 15 may be folded adjacent the first portions 3, 8. Preferably the first and second portions are in fixed engagement. The engagement surfaces of the strap portions (3 & 5 Figure 2 or 8 & 11 Figure 3) may be fixedly engaged by any suitable engagement means such as Velcro™; adhesive, hook, snap and belt holes. A hook or snap, for example, may be located in one or more particular positions along the strap.

The strap 15 may incorporate at least one strap length delimiting means (2 & 6 Figure 2 or 10 & 12 Figure 3). Preferably, there are two length-delimiting means. A strap delimiting means may be fixed in position or may be adjustable in relation to the strap. Preferably, an adjustable strap length-delimiting means moves in a sliding action along the axis of the strap. A delimiting means in fixed position may be a thickened portion of strap or it may be an object such as a stud, lug or the like engaged with the strap.

A strap delimiting means shown as a stud in this embodiment (6 Figure 2 or 9 & 12 Figure 3), may be assembled to, penetrate, or be meshed with, the strap 15 in the second portion (5 Figure 2 or 11 Figure 3). The strap delimiting means is shaped or sized to prevent disengagement or unthreading, partial or otherwise, of the strap and mask frame or attachment. Embodiments of the strap delimiting means may include, without limitation, folding and sewing, bonding or gluing the edge of the strap to have a differently sized or shaped end, such as a thicker or longer end. The strap slide delimiting means (6 Figure 2 or 9 & 12 Figure 3) may be thicker than the aperture or slot (4 Figure 2 or 13 Figure 3), thus preventing the strap from disengaging itself from the mask frame (1 Figure 2 or 7 Figure 3). This would most advantageously avoid the situation where the strap unthreads and a user may have to fumble to rethread the strap end into the mask frame (1 Figure 2 or 7 Figure 3) when trying to re-fit the mask, in the dark, during the middle of a night's sleep. Most advantageously, this invention enables a user to manipulate the strap without seeing it.

Preferably, the strap includes a second strap adjustable delimiting means (2 Figure 2 or 10 Figure 3), which is designed to enable adjustment along the outside surface strap 15, but has enough friction or resistance to prevent undesired movement. Another means of preventing undesired movement, while enabling a sliding feature for optimal configuration, may be a locking mechanism, such as a. spring-based stopper. Preferably, the strap slide limiter, is thicker or longer than the slot (4 Figure 2 or 13 Figure 3), and is set during mask fitting, at a sleep clinic for instance, and then acts as an "optimal setting memory". The optimal setting memory most advantageously operates, when the user pulls the strap while putting the mask on and fastening the straps. The strap slide limiter prevents the strap (3 Figure 2 or 8 Figure 3) from sliding past the optimal setting marker; the strap slide limiter (2 Figure 2 or 10 Figure 3) when it comes in contact with the mask frame (1 Figure 2 or 7 Figure 3) and slot (4 Figure 2 or 13 Figure 3) or a different limiter such as 10 Figure 3.

## Claims

1. A headgear for a mask assembly for the provision of gases to subjects, the headgear comprising a strap (15) for positioning the mask assembly on a user's head; the mask assembly including an aperture (4) through which the strap (15) is passable, in use; the strap (15) including first (3) and second (5) adjacent portions, the second portion being folded against the first portion, in use, wherein the second (5) portion is threadable through the aperture (4); the first (3) and second (5) portions each including a surface for engagement with the complementary surface of the other adjacent portion; **characterised in that** the headgear further comprises a strap length delimiting means (2) on the first portion that moves in a sliding action along an axis of the strap such that the strap length delimiting means is adjustable in relation to the strap, but has enough friction or resistance to prevent undesired movement, and is thicker or longer than the aperture (4) such that, in use, the strap length delimiting means acts as an optimal setting memory which operates when the user pulls the second portion of the strap while putting the mask assembly on and fastening the straps to prevent the first portion (3) from sliding past an optimal setting marker when the strap length delimiting means comes into contact with the aperture (4).

2. The headgear of claim 1, wherein the length delimiting means is a locking mechanism for preventing undesired movement while enabling a sliding feature for optimal configuration.

3. The headgear of claim 2, wherein the locking mechanism comprises a spring-based stopper.

4. The headgear of claim 1, further comprising another strap delimiting means (6, 9, 12) having a stud or a lug or a thickening of the second portion (5) of the strap (15).

5. A method for positioning the headgear for a mask assembly for the provision of gases to subjects according to claim 1, comprising the steps of: placing the headgear on the subject's head; fitting said headgear; and adjusting the fit of said headgear with a length delimiting means.

## Patentansprüche

1. Kopfbedeckung für eine Maskenanordnung für die Versorgung von Personen mit Gasen, wobei die Kopfbedeckung einen Riemen (15) umfasst, um die Maskenanordnung am Kopf eines Benutzers zu positionieren, wobei die Maskenanordnung eine Öffnung (4) umfasst, durch die der Riemen (15) im Gebrauch hindurchführbar ist, wobei der Riemen (15) einen ersten (3) und einen zweiten (5) benachbarten Bereich umfasst, wobei der zweite Bereich im Gebrauch gegen den ersten Bereich gefaltet ist, wobei der zweite Bereich (5) durch die Öffnung (4) hindurchführbar ist, wobei der erste (3) und der zweite (5) Bereich jeweils eine Fläche für einen Eingriff mit einer komplementären Fläche des anderen benachbarten Bereichs aufweisen, **dadurch gekennzeichnet, dass** die Kopfbedeckung weiterhin Mittel (2) für die Begrenzung der Länge des Riemens an dem ersten Bereich umfasst, die sich in einer gleitenden Bewegung entlang einer Achse des Riemens bewegen, so dass die Mittel zur Begrenzung der Riemenlänge in Bezug auf den Riemen einstellbar sind, aber genügend Reibung oder Widerstand aufweisen, um eine unerwünschte Bewegung zu verhindern und dicker oder länger sind als die Öffnung (4), so dass im Gebrauch die Mittel zur Begrenzung der Riemenlänge als ein optimaler Speicher zur Festlegung dienen, welcher agiert, wenn der Benutzer an dem zweiten Bereich des Riemens zieht, wenn er die Maskenanordnung anlegt und die Riemen befestigt, um zu verhindern, dass der erste Bereich (3) über eine optimale Markierung zur Festlegung hinaus gleitet, wenn die Mittel für die Begrenzung der Riemenlänge mit der Öffnung (4) in Kontakt kommen.

2. Kopfbedeckung nach Anspruch 1, bei der die Mittel zur Begrenzung der Riemenlänge ein Verriegelungsmechanismus sind, um eine unerwünschte Bewegung zu verhindern, wobei eine Gleiteigenschaft geschaffen wird für eine optimale Konfiguration.

3. Kopfbedeckung nach Anspruch 2, bei der der Verriegelungsmechanismus einen Stopper auf Federbasis umfasst.

4. Kopfbedeckung nach Anspruch 1, weiterhin umfassend weitere den Riemen begrenzende Mittel (6, 9, 12), die einen Stift oder eine Öse oder eine Verdickung des zweiten Bereichs (5) des Riemens (15) aufweisen.

5. Verfahren zur Positionierung einer Kopfbedeckung für eine Maskenanordnung für die Versorgung von Personen mit Gasen gemäß Anspruch 1, umfassend die Schritte: Platzieren der Kopfbedeckung auf dem Kopf der Person, Befestigen der Kopfbedeckung und Einstellen der Befestigung dieser Kopfbedeckung mit Mitteln für die Begrenzung der Länge.

## Revendications

1. Casque pour un ensemble masque pour la fourniture de gaz à des sujets, le casque comprenant une sangle (15) pour positionner l'ensemble masque sur la tête d'un utilisateur ; l'ensemble masque comprenant une ouverture (4) à travers laquelle la sangle (15) peut être passée, en utilisation ; la sangle (15) comprenant des première (3) et seconde (5) parties adjacentes, la seconde partie étant repliée contre la première partie, en utilisation, la seconde (5) partie étant apte à être enfilée à travers l'ouverture (4) ; les première (3) et seconde (5) parties comprenant chacune une surface pour un engagement avec la surface complémentaire de l'autre partie adjacente ; **caractérisé par le fait que** le casque comprend en outre un moyen de délimitation de longueur de sangle (2) sur la première partie qui se déplace par une action de coulissement le long d'un axe de la sangle de telle sorte que le moyen de délimitation de longueur de sangle est réglable par rapport à la sangle, mais a une friction ou une résistance suffisante pour empêcher un mouvement non souhaité, et est plus épais ou plus long que l'ouverture (4) de telle sorte qu'en utilisation le moyen de délimitation de longueur de sangle agit comme une mémoire de réglage optimal qui fonctionne lorsque l'utilisateur tire la seconde partie de la sangle tout en mettant l'ensemble masque en place et en fixant les sangles pour empêcher la première partie (3) de coulisser au-delà d'un repère de réglage optimal lorsque le moyen de délimitation de longueur de sangle entre en contact avec l'ouverture (4) .

2. Casque selon la revendication 1, dans lequel le moyen de délimitation de longueur est un mécanisme de verrouillage pour empêcher un mouvement non souhaité tout en permettant une fonction de coulissement pour une configuration optimale.

3. Casque selon la revendication 2, dans lequel le mécanisme de verrouillage comprend une butée à ressort.

4. Casque selon la revendication 1, comprenant en outre un autre moyen de délimitation de sangle (6, 9, 12) ayant un goujon ou un ergot ou un épaississement de la seconde partie (5) de la sangle (15).

5. Procédé pour positionner le casque pour un ensemble masque pour la fourniture de gaz à des sujets selon la revendication 1, comprenant les étapes : placer le casque sur la tête du sujet ; ajuster ledit casque ; et régler l'ajustement dudit casque avec un moyen de délimitation de longueur.
